(19) Europäisches Patentamt

European Patent Office

Office européen des brevets

(11) Numéro de publication: **0 269 516**
A2

(12) # DEMANDE DE BREVET EUROPEEN

(21) Numéro de dépôt: 87402615.6

(22) Date de dépôt: 19.11.87

(51) Int. Cl.⁴: **A 61 K 31/575**

(30) Priorité: 20.11.86 FR 8616139

(43) Date de publication de la demande:
01.06.88 Bulletin 88/22

(84) Etats contractants désignés:
AT BE CH DE FR GB IT LI LU NL SE

(71) Demandeur: SYNTHELABO
58 rue de la Glacière
F-75013 Paris (FR)

(72) Inventeur: Poupon, Raoul
63, rue du Chemin Vert
F-75011 Paris (FR)

(74) Mandataire: Thouret-Lemaitre, Elisabeth et al
Service Brevets - SYNTHELABO 58, rue de la Glacière
F-75621 Paris Cédex 13 (FR)

(54) Compositions pharmaceutiques contenant de l'acide ursodesoxycholique.

(57) Utilisation de l'acide ursodesoxycholique pour la fabrication de moyens destinés au traitement de la cirrhose biliaire primitive.

EP 0 269 516 A2

Bundesdruckerei Berlin

# Description

## COMPOSITIONS PHARMACEUTIQUES CONTENANT DE L'ACIDE URSODESOXYCHOLIQUE

La présente invention a pour objet l'utilisation de l'acide ursodesoxycholique pour la fabrication de moyens destinés au traitement de la cirrhose biliaire primitive.

L'acide ursodesoxycholique est un composé connu utilisé pour le traitement dissolvant de la lithiase biliaire cholestérolique.

La Demanderesse a pu constater que l'acide ursodesoxycholique agit également efficacement dans le traitement de la cirrhose biliaire primitive.

La cirrhose biliaire primitive est une maladie mortelle.

La cirrhose biliaire primitive est dite primitive car elle n'est consécutive ni à un abus d'alcool, ni à une hépatite toxique ou infectieuse. Elle touche surtout la femme d'âge mûr (40-50 ans). C'est une affection due à la destruction progressive des petits canaux biliaires intra-hépatiques.

L'évolution est de durée variable : de 5 à 10 ans en moyenne. Elle se manifeste par une fatigue, des démangeaisons, puis par un gros foie et un ictère. La seule chance de survie à long terme est représentée actuellement par la transplantation hépatique.

La mise en évidence d'anticorps anti-mitochondries chez ces malades accréditait jusqu'à présent l'hypothèse d'une maladie auto-immune. C'est pourquoi tous les traitements utilisés sont encore de type immuno-suppresseur : corticoïdes à fortes doses, azathioprine. La démarche entreprise avec l'acide ursodésoxycholique repose sur l'hypothèse que les manifestations et la gravité de la maladie sont dues, non pas à un désordre immunologique, mais à la toxicité de certaines substances synthétisées par le foie : les acides biliaires. Ces acides participent à la digestion des lipides. Ils subissent un cycle métabolique en circuit fermé : élimination par la bile et réabsorption dans l'intestin.

Administré en continu, l'acide ursodésoxycholique, qui est, lui, d'une totale inocuité, se substitue physiologiquement à ces acides et permet leur élimination de l'organisme.

Après administration pendant 2 ans de 13 à 15 mg/kg/jour d'acide ursodesoxycholique à des patients, la concentration totale des acides biliaires sériques n'a pas été modifiée ; le pourcentage de malades ayant un prurit est passé de 53 à 8 %, la bilirubinémie des patients est devenue inférieure à 34 μM, les activités sériques des phosphatases alcalines, des transaminases et de la γ -glutamyl transférase ont diminué chez tous les patients, les taux de prothrombine, d'albumine, de γ -globulines et d'immunoglobulines M n'ont pas été modifiés.

Après un recul de plusieurs années (5 ans) il y a confirmation des résultats obtenus ; le nombre de malades traités montre que l'effet thérapeutique augmente et qu'il n'y a pas d'effets secondaires.

Les compositions pharmaceutiques contenant l'acide ursodesoxycholique en association avec tout excipient approprié font partie de l'invention. Elles sont administrées de préférence par voie orale.

Un exemple de composition pharmaceutique sous forme de gélule est le suivant
- acide ursodesoxycholique 200 mg
- excipient : stéarate de magnésium, talc, amijel, amidon de maïs
- gélule : gélatine, bioxyde de titane, indigotine, anhydride sulfureux.

## Revendications

Utilisation de l'acide ursodesoxycholique pour la fabrication de moyens destinés au traitement de la cirrhose biliaire primitive.